# EUROPÄISCHE PATENTANMELDUNG

(11) **EP 4 008 294 A1**
(43) Veröffentlichungstag der Anmeldung: **08.06.2022**
(21) Anmeldenummer: 21210347.7
(22) Anmeldetag: 25.11.2021
(51) Int. Cl.: A61F 5/02

(54) **RÜCKENORTHESE UND KIT FÜR EINE RÜCKENORTHESE**

(30) Priorität: 03.12.2020 DE 102020132128
(71) Anmelder: Bort GmbH, 71384 Weinstadt-Benzach (DE)
(72) Erfinder: Freihaut, Roman, 73614 Schorndorf (DE)
(74) Vertreter: Thum, Bernhard

(57) **Zusammenfassung**

Die Erfindung betrifft eine Rückenorthese (10) zum Stützen der Lenden- oder/und Brustwirbelsäule. Die Rückenorthese umfasst ein flexibles Rückenelement (12), das dazu ausgelegt ist, in einem Tragezustand an einem Rücken eines Benutzers anzuliegen, einen Stützkörper (16), der an die menschliche Anatomie der Wirbelsäule angepasste oder anpassbare geschwungene Bereiche aufweist und in dem Rückenelement (12) aufgenommen ist, und ein Befestigungssystem (14), das mit dem Rückenelement (12) verbunden ist und dazu ausgelegt ist, in dem Tragezustand das Rückenelement (12) anpassbar an dem Benutzer anzubringen. Ferner umfasst die Rückenorthese (10) wenigstens einen Versteifungskörper (18), der verstellbar an dem Stützkörper (16) angebracht oder anbringbar ist. Zudem betrifft die Erfindung einen Kit für eine Rückenorthese (10), der eine Mehrzahl an Versteifungskörpern (18) mit unterschiedlicher Steifigkeit oder/und unterschiedlicher Länge umfasst.

## Beschreibung

Die vorliegende Erfindung betrifft eine Rückenorthese zum Stützen der Lenden- oder/und Brustwirbelsäule. Die Rückenorthese umfasst ein flexibles Rückenelement, das dazu ausgelegt ist, in einem Tragezustand an einem Rücken eines Benutzers anzuliegen, einen Stützkörper, der an die menschliche Anatomie der Wirbelsäule angepasste oder anpassbare geschwungene Bereiche aufweist und in dem Rückenelement aufgenommen ist, und ein Befestigungssystem, das mit dem Rückenelement verbunden ist und dazu ausgelegt ist, in dem Tragezustand das Rückenelement anpassbar an dem Benutzer anzubringen.

Mittels einer aktiven Aufrichtung zur Entlastung und Korrektur der Lendenwirbelsäule (LWS) und Brustwirbelsäule (BWS) können Bewegungseinschränkungen in der Sagittalebene gemildert oder behoben werden. Derartige Bewegungseinschränkungen können beispielsweise verursacht sein durch Wirbelkörperfrakturen, Osteoporose, eine konservative Therapie sekundärer Kyphosen oder muskuläre Insuffizienz. Aber auch ein Rundrücken mit Schulterfehlhaltung und Erkrankungen des rheumatischen Formenkreises mit chronischen Rückenschmerzen, erosive Chondrosen und Vertebrostenosen können mit einer aktiven Aufrichtung der LWS und BWS behandelt werden.

Rückenorthesen zur Behandlung vorstehender Bewegungseinschränkungen können von einem Benutzer, also von einem zu behandelnden Patienten, an seinem/ihrem Rücken getragen werden. Dabei unterstützt die Rückenorthese die LWS und BWS, sodass der Rücken des Benutzers entlastet wird.

Eine solche Rückenorthese ist beispielsweise aus EP 1 834 613 B9 bekannt. Die Rückenorthese ist als ein Baukastensystem angedacht, das mehrere Stützelemente umfasst, die je nach Beschwerden und Genesungszustand des Patienten kombiniert werden können. Auf diese Weise ist die Rückenorthese an unterschiedliche Beschwerden und Genesungszustände eines Patienten anpassbar. Nachteilig ist jedoch, dass die Stützelemente sehr sperrig sind und somit die gesamte Rückenorthese eher unhandlig und wenig optisch ansprechend ist.

Eine andere Möglichkeit der flexiblen Anpassung von Stützeigenschaften einer LWS-Orthese ist aus US 9,636,247 bekannt. Danach wird ein Stützelement in eine Tasche der Orthese eingesetzt, das einen ersten Stützkörper und einen zweiten Stützkörper umfasst. Die beiden Stützkörper sind miteinander verschraubbar, wobei durch die Position der Verschraubung der beiden Stützkörper miteinander eine Lordosestützfunktion bzw. eine Biegung des ersten Stützkörpers einstellbar ist.

Eine ergonomische und optisch ansprechende Rückenorthese ist die von der Fa. Bort vertriebene "BORT Generation Osteoporose-Orthese". Diese Rückenorthese weist eine platzsparende und anatomisch geformte Rückenplatte zur aktiven Entlastung und Korrektur der LWS und BWS auf. Die zur Entlastung der LWS und RWS vorgesehene Rückenplatte ist in einem Rückenelement aufgenommen, das mittels eines Gurtsystems an der gewünschten Position an dem Rücken des Patienten gehalten ist. Dazu läuft im Tragezustand der Rückenorthese das Gurtsystem um die Schultern des Benutzers herum, wird im Bereich der Taille umgelenkt und dann im Bereich der Hüfte fixiert. Mittels einer Anpassung der Beugung der Rückenplatte kann die Rückenorthese an den Benutzer angepasst werden. Jedoch ist eine Anpassung an unterschiedliche Beschwerden und Genesungszustände eines Patienten nur in einem geringen Maße möglich.

Aufgrund der Vielzahl der zu behandelnden Bewegungseinschränkungen, die sich meist sowohl auf die LWS als auch die BWS auswirken, ist es erstrebenswert, eine Rückenorthese bereitzustellen, die beide der genannten Wirbelsäulenbereiche gesamtheitlich unterstützen kann, aber dennoch eine individuelle Anpassung einzelner Wirbelsäulenbereiche an die unterschiedlichen Beschwerden und Genesungszustände des jeweiligen Patienten ermöglicht.

Es ist daher die Aufgabe der vorliegenden Erfindung, eine Rückenorthese zur Stützung der Lendenwirbelsäule und Brustwirbelsäule bereitzustellen, die variabel an den Stützbedarf der Wirbelsäule eines Patienten anpassbar ist.

Diese Aufgabe wird erfindungsgemäß durch eine Rückenorthese der eingangs bezeichneten Art gelöst, bei der vorgesehen ist, dass die Rückenorthese ferner wenigstens einen Versteifungskörper umfasst, der verstellbar an dem Stützkörper angebracht oder anbringbar ist.

Die erfindungsgemäße Rückenorthese ermöglicht es, sowohl die Lendenwirbelsäule (LWS) als auch die Brustwirbelsäule (BWS) zu stützen. Dazu weist der Stützkörper an die menschliche Anatomie der Wirbelsäule angepasste, geschwungene Bereiche auf. Beispielsweise weist der Stützkörper zwei entgegengesetzt geschwungene Bereiche für die Anpassung an die menschliche Anatomie der Wirbelsäule auf. Zudem kann der Stützkörper verformbar sein, um gezielt an die jeweilige Krümmung der Wirbelsäule eines Benutzers angepasst zu werden. Die Verformung von Stützkörper und Versteifungskörper kann durch im Gebiet der Orthopädie ausgebildetes Fachpersonal oder durch den Benutzer selbst erfolgen. Die Verformung kann durch Muskelkraft und manuell bedienbares Werkzeug erfolgen.

Häufig sind einzelne Wirbelsäulenbereiche eines Benutzers bzw. Patienten stärker belastet als andere. Mit dem Stützkörper werden alle Wirbelsäulenbereiche im Wesentlichen gleich unterstützt. Einzelne Bereiche der LWS und BWS, bei denen eine größere Unterstützung gewünscht ist, können mittels des wenigstens einen Versteifungskörpers zudem ganz gezielt unterstützt werden. Dazu kann der wenigstens eine Versteifungskörper an dem Stützkörper derart positioniert und angebracht werden, dass er im Tragezustand den gewünschten Bereich der Wirbelsäule unterstützt. Auch kann die Position des Versteifungskörpers im Laufe der Behandlungszeit, in der der Benutzer die Rückenorthese trägt, verändert werden. Der oder die Abschnitte des Stützkörpers, an dem/denen der wenigstens eine Versteifungskörper angebracht ist, führen zu einer lokalen Versteifung und einer lokalen Erhöhung der Stützeigenschaften der Rückenorthese. Der wenigstens eine Versteifungskörper bestimmt folglich das Ausmaß der lokalen Versteifung bzw. Verstärkung des Stützkörpers. Somit trägt die Rückenorthese nicht nur zur Stützung der LWS und BWS bei, sondern kann auch zur aktiven Aufrichtung, Entlastung oder/und Korrektur der Lendenwirbelsäule und Brustwirbelsäule beitragen.

Da der wenigstens eine Versteifungskörper verformbar und an dem Stützkörper individuell positionierbar ist, kann dessen Position an dem Stützkörper bei einem Behandlungsfortschritt oder einen sich ändernden Bedarf einer Stützung der LWS und BWS angepasst werden. Es kann lediglich ein Versteifungskörper an dem Stützkörper angebracht sein. Alternativ können auch mehrere Versteifungskörper an den Stützkörper angebracht werden, um gezielt mehrere Bereiche der LWS und BWS zu stützen. Auf diese Weise kann der Grad der Stützung individuell und angepasst an die jeweilige Behandlung erfolgen.

Der Stützkörper kann eine Prägung aufweisen, die den Bereich markiert, an dem die Versteifungsplatte, vorzugsweise abhängig von der Diagnose, an dem Stützkörper zu positionieren ist. Die Prägung kann Bereiche anzeigen, die häufig frakturiert sind. Es wird also eine Frakturprävalenz berücksichtigt. Dadurch kann das Risiko einer Fehlpositionierung vermindert werden.

Der Stützkörper oder/und der wenigstens eine Versteifungskörper kann/können aus Aluminium hergestellt sein. Aluminium hat die gewünschte Festigkeit zur Stützung der LWS und BWS und ist zudem mit verhältnismäßig geringer Kraft verformbar, sodass der Stützkörper bzw. der Versteifungskörper mit verhältnismäßig wenig Kraftaufwand an die menschliche Anatomie der LWS und BWS des Benutzers anpassbar ist.

Für einen angenehmen Tragekomfort und eine optisch ansprechende Gestaltung der Rückenorthese kann der Stützkörper oder/und der wenigstens eine Versteifungskörper beispielsweise vollständig in dem Rückenelement aufgenommen sein.

Der wenigstens eine Versteifungskörper kann gemäß einer Weiterbildung der Erfindung dazu ausgelegt sein, formschlüssig an einem Abschnitt des Stützkörpers anzuliegen. Das Anliegen kann flächig, punktuell oder abschnittsweise sein. Auf diese Weise kann eine effektive Änderung der Stützung der Wirbelsäule des durch den Versteifungskörper versteiften bzw. verstärkten Bereichs erzielt werden. Auch ist eine formschlüssige Verbindung zwischen Stützkörper und wenigstens einem Versteifungskörper sehr platzsparend, wodurch die Rückenorthese optisch ansprechend gestaltet ist.

Es kann erfindungsgemäß ferner vorgesehen sein, dass der wenigstens eine Versteifungskörper dazu ausgelegt ist, mittels wenigstens einem Sicherungselement an dem Stützkörper gesichert zu sein. Somit wird ein Verschieben des Versteifungskörpers gegenüber dem Stützkörper in Längs- oder/und Querrichtung vermieden.

Der Abschnitt des Stützkörpers, an dem der wenigstens eine Versteifungskörper gesichert ist, kann gemäß einer Weiterbildung der Erfindung frei wählbar sein. Der wenigstens eine Versteifungskörper kann dorsal an dem Stützkörper angebracht oder anbringbar sein. Auf diese Weise kann eine individuelle Anpassung der Rückenorthese an die für die Behandlung benötigte Stützung erfolgen.

In einer Weiterbildung der Erfindung kann die Rückenorthese wenigstens zwei Versteifungskörper umfassen, die unabhängig voneinander oder miteinander verbunden an dem Stützkörper angebracht oder anbringbar sind. Es versteht sich, dass mittels der Verwendung mehrerer Versteifungskörper mehrere Abschnitte der Wirbelsäule stärker gestützt werden können. Bei fortschreitender Behandlung können auch einzelne Versteifungskörper verschoben, neu positioniert oder/und wieder entfernt werden.

Der Stützkörper und der wenigstens eine Versteifungskörper können gemäß einer Weiterbildung der Erfindung eine Mehrzahl an Öffnungen aufweisen, die jeweils entlang des Stützkörpers voneinander beabstandet im Wesentlichen mittig angeordnet sind. Die Öffnungen des Stützkörpers und des Versteifungskörpers können gleich dimensioniert und geformt sein.

Zudem können der Stützkörper und der wenigstens eine Versteifungskörper gemäß einer Weiterbildung der Erfindung derart gegeneinander verschiebbar sein, dass wenigstens eine Öffnung des Stützkörpers mit einer Öffnung des wenigstens einen Versteifungskörpers eine gemeinsame Durchgangsöffnung bilden. Die Öffnungen tragen einerseits zur Gewichtsreduzierung der Rückenorthese bei und können andererseits Teil der Sicherung des wenigstens einen Versteifungskörpers an dem Stützkörper sein, wie im Folgenden genauer beschrieben ist.

Gemäß einer Ausführungsvariante der Erfindung ist vorgesehen, dass die Sicherungselemente ein Verbindungselement, zum Beispiel ein Klammerelement, Klemmen, Klemmschellen oder eine Schraubvorrichtung, umfassen. Das Verbindungselement ist beispielsweise dazu ausgebildet, ein Verschieben des Versteifungskörpers gegenüber dem Stützkörper, insbesondere entlang der Längsrichtung davon, zu verhindern.

Das Verbindungselement kann gemäß einer Weiterbildung der Erfindung derart ausgebildet sein, dass es den Stützkörper und den Versteifungskörper seitlich umgreift oder/und die gemeinsame Durchgangsöffnung durchtritt, sodass der Stützkörper und der wenigstens eine Versteifungskörper aneinander gesichert sind. Mittels des Verbindungselements kann also eine den Stützkörper und den Versteifungskörper umschließende Fixierung erfolgen.

Das Verbindungselement kann gemäß einer Weiterbildung der Erfindung zwei elastisch oder schwenkbar miteinander verbundene Verbindungsabschnitte und einen Verrastabschnitt umfassen, sodass das Verbindungselement nach Umgreifen von dem Stützkörper und dem Versteifungskörper mittels dem Verrastabschnitt verrastet werden kann. Das Verbindungselement ist beispielsweise so dimensioniert, dass dessen Innenabmessungen lediglich geringfügig größer sind als die Außenabmessungen des Stützkörpers und des Versteifungskörpers zusammen, wenn diese aneinander anliegen. Es liegt also bevorzugt zumindest teilweise ein Formschluss zwischen Stützkörper, Versteifungskörper und Verbindungselement vor.

Beispielsweise werden mehrere Verbindungselemente verwendet, um eine hinreichende Sicherung des wenigstens einen Versteifungskörpers an dem Stützkörper zu erzielen. Vorzugsweise werden wenigstens zwei, weiter bevorzugt drei, ferner bevorzugt vier oder mehr Verbindungselemente verwendet, um den wenigstens einen Versteifungskörper an dem Stützkörper zu sichern. Durch die Verbindungselemente allgemein und insbesondere durch die Verwendung mehrerer Verbindungselemente kann ein laterales Verschieben ebenso wie ein Verschieben bzw. Verrutschen in Längsrichtung des Versteifungskörpers relativ zu dem Stützkörper vermieden werden.

Zur Erzielung der gewünschten Stützung der LWS oder/und BWS ist es ferner von Vorteil, wenn die Rückenorthese an dem Benutzer so angebracht werden kann, dass sich die gewünschten Stützeigenschaften ergeben. Zu diesem Zweck kann das Befestigungssystem zum Befestigen des Rückenelements ein Gurtsystem aufweisen, das einen ersten und einen zweiten Gurtstrang umfasst, wobei jeder der zwei Gurtstränge an zwei Befestigungsstellen und einer Umlenkstelle an dem Rückenelement befestigt ist.

Das Grundmaterial der Gurtstränge ist gemäß einer Weiterbildung der Erfindung beispielsweise ein biegsames, flexibles, nicht oder nur geringfügig elastisches Material, z.B. ein Stoffmaterial oder eine Materialkombination aus Kunststoff- und Stoffmaterialien. Die Materialauswahl ermöglicht ein komfortables Tragen der Rückenorthese.

Ferner trägt zu einem komfortablen Tragen der erfindungsgemäßen Rückenorthese bei, wenn eine erste der zwei Befestigungsstellen des ersten Gurtstrangs an einem linken ersten Ende des Rückenelements angeordnet ist, das im Tragezustand sinister ist, und eine zweite der zwei Befestigungsstellen des ersten Gurtstrangs an einem linken zweiten Ende des Rückenelements angeordnet ist, das im Tragezustand sinister ist. Die Umlenkstelle des ersten Gurtstrangs kann an einem mittleren Bereich des Rückenelements angeordnet sein.

Vorzugsweise kann die Rückenorthese gemäß der Erfindung im Wesentlichen, abgesehen von eventuellen Gurtstrangbefestigungssystemen, spiegelsymmetrisch zur Wirbelsäule bzw. zu einer vertikal verlaufenden Ebene ausgebildet sein.

Daher kann eine erste der zwei Befestigungsstellen des zweiten Gurtstrangs an einem rechten ersten Ende des Rückenelements angeordnet sein, das im Tragezustand dexter ist, eine zweite der zwei Befestigungsstellen des zweiten Gurtstrangs an einem rechten zweiten Ende des Rückenelements angeordnet sein, das im Tragezustand sinister ist, und die Umlenkstelle des zweiten Gurtstrangs an einem mittleren Bereich des Rückenelements angeordnet sein.

Es versteht sich, dass eine Länge der Gurtstränge mit bekannten Schnallensystemen einstellbar ist. Auch können die zwei Gurtstränge dazu ausgelegt sein, ventral miteinander verbunden zu werden, um die Rückenorthese an dem Benutzer in der gewünschten Position anzulegen.

Im Tragezustand der erfindungsgemäßen Rückenorthese kann der Benutzer verschiedene Bewegungen, wie zum Beispiel Drehbewegungen des Oberkörpers oder Bewegungen der Schultern ausführen. Um in allen diesen Bewegungssituationen ein angenehmes Tragegefühl bereitzustellen, kann jeder der beiden Gurtstränge wenigstens einen elastischen Abschnitt aufweisen.

Gemäß einer Ausführungsvariante der Erfindung ist vorgesehen, dass ein jeweiliger mittlerer elastischer Abschnitt des ersten bzw. zweiten Gurtstranges in dem mittleren Bereich angeordnet ist, wo der jeweilige Gurtstrang umgelenkt wird. Im Tragezustand ist der mittlere Bereich in etwa benachbart der Taille des Benutzers angeordnet. Der elastische Abschnitt im mittleren Bereich kann somit hinreichend Bewegungsspielraum bereitstellen, dass der Benutzer ohne oder mit lediglich wenig Einschränkung seinen Oberkörper drehen kann.

Zusätzlich oder alternativ kann gemäß einer Weiterbildung der Erfindung ein jeweiliger elastischer Schulterabschnitt des ersten bzw. zweiten Gurtstranges in dem Tragezustand benachbart der Schulter eines Benutzers der Rückenorthese angeordnet sein. Im Tragezustand kann der elastische Schulterabschnitt in einem ventralen Bereich des Schultergurts angeordnet sein. Auf diese Weise kann im Tragezustand die Bewegungsfreiheit im Schulterbereich des Oberkörpers des Benutzers gegenüber Gurtsträngen ohne elastischen Schulterabschnitt erhöht werden. Auch können kleine Bewegungsausschläge, die im Schulterbereich entstehen, mit dem elastischen Schulterabschnitt ausgeglichen werden, ohne dass die Gurtstränge den Benutzer in seiner Bewegungsfreiheit einschränken, wodurch die Rückenorthese komfortabler zu tragen ist.

Mittels der elastischen Abschnitte ist somit die erfindungsgemäße Rückenorthese insgesamt weniger regide, also weniger starr, und somit flexibler und angenehmer zu tragen im Vergleich zu Gurtsystemen ohne elastische Abschnitte. In einigen Fällen kann es jedoch aus medizinischer Sicht wünschenswert sein, wenn die genannte Bewegungsfreiheit eingeschränkt ist. Daher kann jeder der elastischen Schulterabschnitte von einem jeweiligen Begrenzungszügel überspannt sein, der nicht elastisch sowie abnehmbar ist, sodass der jeweilige Gurtstrang im Bereich des linken bzw. rechten Schulterabschnitts nur dann flexibel ist, wenn der jeweilige Begrenzungszügel abgenommen ist. Anders ausgedrückt, eine Erhöhung der dynamischen Eigenschaften des Gurtsystems der Rückenorthese wird erzielt, indem die Begrenzungszügel abgenommen werden. Die Begrenzungszügel können auf den jeweiligen Gurtstrang aufgeklettet sein.

Nach einem weiteren Aspekt der Erfindung wird ein Kit für eine Rückenorthese bereitgestellt, die wie vorstehend ausgeführt ist, und eine Mehrzahl an Versteifungskörpern mit unterschiedlicher Steifigkeit oder/und unterschiedlicher Länge umfasst. Auf diese Weise kann die Rückenorthese ganz individuell an den jeweiligen Benutzer und dessen Behandlungsziel und anatomische Besonderheiten der LWS und BWS angepasst werden.

Alle der vorstehend erläuterten Anpassungsmöglichkeiten der Rückenorthese mittels elastischen Abschnitten und einer Auswahl aus verschiedenen Versteifungskörpern können die dynamischen Komponenten, das Biofeedback und die Individualisierung der Patientenversorgung verbessern.

Im Folgenden werden verschiedene Ausführungsformen der Erfindung beispielhaft anhand der beiliegenden Figuren erläutert. Es stellen dar:
- Figuren 1a-b: eine Draufsicht auf eine Rückenorthese, bei der der Stützkörper und ein Begrenzungszügel abgenommen sind, gemäß einer Ausführungsform der Rückenorthese;
- Figur 2: eine Seitenansicht auf einen Stützkörper und einen Versteifungskörper sowie eine Draufsicht auf Sicherungselemente;
- Figuren 3a-b: eine Draufsicht auf einen Stützkörper (Figur 3a) und auf mit dem Stützkörper mittels Sicherungselementen verbundene Versteifungskörper (Figur 3b);
- Figuren 4a-b: eine Verdeutlichung von verschiedenen Anbringungspositionen zweier Versteifungskörper an dem Stützkörper mittels des Sicherungselements gemäß einer ersten Ausführungsform;

- Figuren 5a-g: eine Verdeutlichung des Ablaufs zur Verbindung des Stützkörpers mit dem Versteifungskörper mittels des Sicherungselements gemäß der ersten Ausführungsform; und
- Figuren 6a-b: perspektivische Ansichten eines Sicherungselements, das gemäß einer zweiten Ausführungsform ausgebildet ist, in einer den Stützkörper und den Versteifungskörper sichernden Position (Figur 6a) und in einer Einzeldarstellung (Figur 6b).

### Figurenbeschreibung

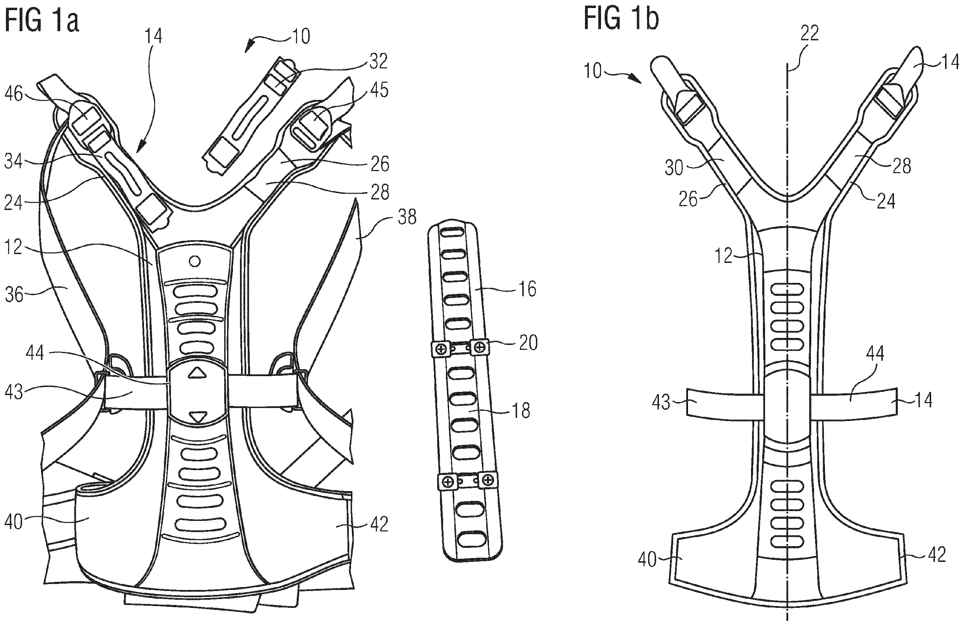

In Figur 1a ist ein Ausführungsbeispiel einer erfindungsgemäßen Rückenorthese in einer Draufsicht gezeigt und allgemein mit 10 bezeichnet. Die Rückenorthese 10 umfasst ein flexibles Rückenelement 12, ein Befestigungssystem 14 und einen Stützkörper 16, der in dem flexiblen Rückenelement 12 aufnehmbar ist, jedoch in der Darstellung von Figur 1a aus diesen entnommen und danebenliegend gezeigt ist. Ferner umfasst die Rückenorthese 10 einen Versteifungskörper 18, der in der Darstellung von Figur 1a an dem Stützkörper 16 mittels Sicherungselementen 20 gesichert ist.

Die Rückenorthese 10 ist dazu ausgelegt, dorsal an einem Rücken eines Benutzers anzuliegen und mittels des Befestigungssystems 14, das in der Darstellung von Figur 1a als Gurtsystem dargestellt ist, an den Benutzer angelegt zu werden. Bevorzugt ist in einem Tragezustand, in dem der Benutzer die Rückenorthese 10 trägt, die Rückenorthese 10 zu der Wirbelsäule des Benutzers ausgerichtet. Beispielsweise kann die in Figur 1b dargestellte Symmetrieebene 22 durch das Rückenelement zu der Wirbelsäule des Benutzers ausgerichtet sein, wobei das Rückenelement 12 symmetrisch zu der Symmetrieebene 22 ausgebildet ist.

An das Rückenelement 12 schließen sich an einem ersten Ende zwei Schulter-Gurtstränge 24, 26 an, von denen im Tragezustand der eine 24 sinister (links) und der andere 26 dexter (rechts) angeordnet ist. Diese beiden Schulter-Gurtstränge 24, 26 sind dazu ausgebildet, im Tragezustand an einer Schulter des Benutzers anzuliegen. Wie in Figuren 1a-b ersichtlich, können die Schulter-Gurtstränge 24, 26 jeweils einen elastischen Schulterabschnitt 28, 30 umfassen, dessen Elastizität größer ist als ein anderer nicht-elastischer Abschnitt der Schulter-Gurtstränge 24, 26. Die elastischen Schulterabschnitte 28, 30 ermöglichen eine elastische Verformung, beispielsweise eine Ausdehnung in Längsrichtung, der Schulter-Gurtstränge 24, 26, wenn eine Zugkraft auf diese ausgeübt wird. Die elastischen Abschnitte 28, 30 sind von einem Begrenzungszügel 32, 34 überspannt, der dazu ausgelegt ist, derart an nichtelastischen Abschnitten der Schulter-Gurtstränge 24, 26 anzuliegen, dass der elastische Abschnitt bei Aufbringen einer Zugkraft auf die Schulter-Gurtstränge 24, 26 keine oder nur eine geringe elastische Verformung erfährt. Im Tragezustand können die elastischen Schulterabschnitte 28, 30 benachbart der Schulter, beispielsweise auf der Schulter oder dorsal, angeordnet sein.

Die Begrenzungszügel 32, 34 sind von dem Befestigungssystem 14 abnehmbar, wobei in Figur 1a einer 32 der beiden Begrenzungszügel 32, 34 in einem von dem Befestigungssystem 14 gelösten Zustand dargestellt ist, während der andere 34 an dem Befestigungssystem 14 befestigt ist. Die Befestigung der Begrenzungszügel 32, 34 kann über aus dem Stand der Technik bekannte Befestigungsverfahren erfolgen, wie beispielsweise mittels eines Klettverschlusses.

Jeder der Schulter-Gurtstränge 24, 26 geht in einen jeweiligen umgelenkten Gurtstrang 36, 38 über. Der umgelenkte Gurtstrang 36, 38 ist in einem mittleren Bereich des flexiblen Rückenelements 12 mit diesem verbunden und wird in diesem mittleren Bereich umgelenkt. Der mittlere Bereich des flexiblen Rückenelements 12 kann dazu ausgelegt sein, im Tragezustand benachbart der Taille des Benutzers angeordnet zu sein.

Jeder der umgelenkten Gurtstränge 36, 38 geht in einen Hüft-Gurtstrang 40, 42 über, der an einem zweiten Ende des flexiblen Rückenelements mit diesem verbunden ist. Die Hüft-Gurtstränge 40, 42 können dazu ausgelegt sein, im Tragezustand benachbart der Hüfte des Benutzers angeordnet zu sein. Auch wenn dies in den Figuren nicht ersichtlich ist, kann das Befestigungssystem 14 Befestigungsabschnitte umfassen, die die Hüfte des Benutzers umfänglich umgeben, um die Rückenorthese 10 an der Hüfte des Benutzers zu befestigen.

Ferner können auch die umgelenkten Gurtstränge 38, 40 sowie die Hüft-Gurtstränge 40, 42 elastische Abschnitte umfassen, deren Elastizität größer ist als diejenige anderer Bereiche der jeweiligen Gurtstränge, die keine elastischen Abschnitte umfassen, und die bei Aufbringen einer Zugkraft eine Verlängerung oder eine entsprechende elastische Verformung des jeweiligen Gurtstrangs bewirken. Mittlere elastische Abschnitte 43, 44, die im mittleren Bereich des flexiblen Rückenelements angeordnet sind, sind in Figur 1a ersichtlich. Schnallen 45, 46 zur Einstellung der Länge der Gurtstränge sind beispielhaft in Figur 1b dargestellt.

Im Tragezustand der Rückenorthese 10 kann der Benutzer verschiedene Bewegungen ausführen, wie zum Beispiel Drehbewegungen. Um in allen diesen Bewegungssituationen ein angenehmes Tragegefühl bereitzustellen, kann jeder der beiden Gurtstränge 24, 26, 36, 38, 40, 42 wenigstens einen elastischen Abschnitt aufweisen.

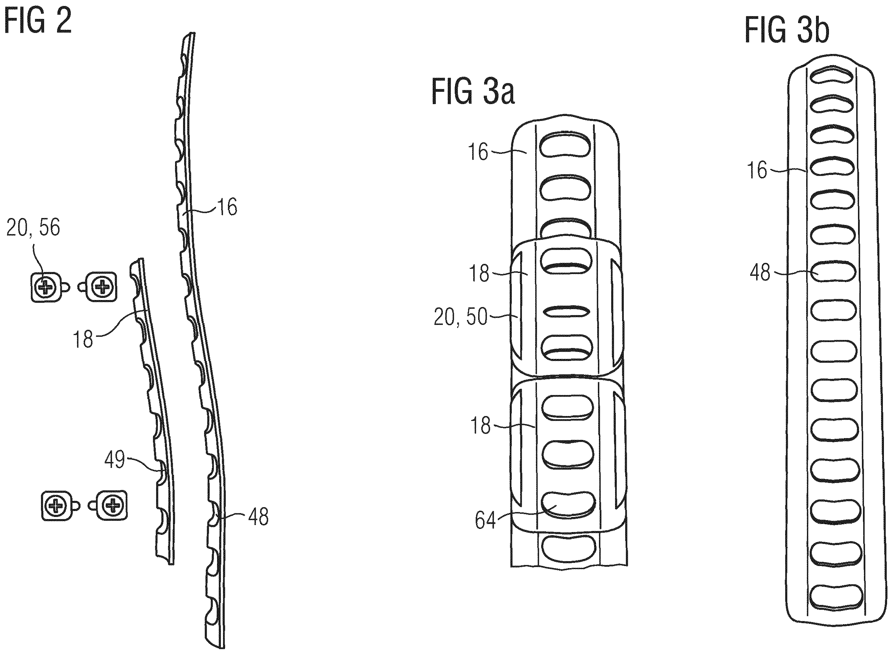

Durch den Stützkörper 16 und wenigstens einem daran angebrachten Versteifungskörper 18, die in dem flexiblen Rückenelement 12 aufgenommen sind, erfolgt die gewünschte Stützung der Lendenwirbelsäule (LWS) oder/und der Brustwirbelsäule (BWS). In der in Figur 2 gezeigten Seitenansicht des Stützkörpers 16 und des Versteifungskörpers 18 sind deren geschwungene und an die menschliche Anatomie der Wirbelsäule angepasste Formen ersichtlich. Die geschwungene Form beider Körper 16, 18 kann an die jeweilige Krümmung der Wirbelsäule des Benutzers angepasst werden. Zur Positionierung des Versteifungskörpers 18 an dem Stützkörper 16 werden diese aneinander angelegt und gegebenenfalls entlang ihrer Längsachse gegeneinander verschoben, bis sich der Versteifungskörper 18 an der gewünschten Position befindet. In dieser Position liegen der Versteifungskörper 18 und der Stützkörper 16 zumindest punktuell oder abschnittsweise formschlüssig aneinander an. Die Fixierung des Versteifungskörpers 18 an dem Stützkörper 16 erfolgt durch Sicherungselemente 20.

Eine gewünschte Versteifung des Stützkörpers 16 mittels des Versteifungskörpers 18 kann an unterschiedlichen Positionen an dem Stützkörper 16 erfolgen, je nachdem an welcher Stelle eine stärkere Stützung der LWS oder/und BWS gewünscht ist, als mit dem Stützkörper 16 allein erzielt werden kann. Auch kann die Länge des Versteifungskörpers 18 an die gewünschte Stützung angepasst sein. Die Länge des Stützkörpers 16 ist vordefiniert, sie kann jedoch an die Rückenlänge des Benutzers angepasst sein. Die Länge des wenigstens einen Versteifungskörpers 18 ist kleiner als die Länge des Stützkörpers 16. Vorzugsweise ist die Länge des Versteifungskörpers länger als halb so lang wie die Länge des Stützkörpers.

Die Abmessungen des Stützkörpers 16 können wie folgt sein: mögliche Längen des Stützkörpers 16 liegen im Bereich zwischen 460 mm und 600 mm, jedoch bevorzugt 540 mm; zwischen 385 mm und 415 mm, jedoch bevorzugt 400 mm; zwischen 445 mm und 475 mm, jedoch bevorzugt 460 mm; oder/und zwischen 505 mm und 535 mm, jedoch bevorzugt 520 mm, eine mögliche Breite des Stützkörpers 16 liegt im Bereich zwischen 68 mm und 78 mm, ist jedoch bevorzugt 73 mm, und eine mögliche Materialdicke des Stützkörpers liegt im Bereich zwischen 1,0 mm und 2,0 mm, ist jedoch bevorzugt 1,5 mm.

Die Abmessungen des Versteifungskörpers 18 können wie folgt sein: eine Länge kann zwischen 150 mm und 500 mm sein, eine Breite kann zwischen 68 mm und 78 mm sein, jedoch bevorzugt 73 mm sein, und eine Materialdicke kann zwischen 1,0 mm und 2,0 mm sein, jedoch bevorzugt 1,5 mm sein. Die Länge des Versteifungskörpers kann beispielsweise abhängig von der gewünschten Stützwirkung bzw. der vorliegenden Diagnose gewählt werden. Mögliche Längen des Versteifungskörpers liegen im Bereich zwischen 179 mm und 209 mm, jedoch bevorzugt 194 mm; zwischen 239 mm und 269 mm, jedoch bevorzugt 254 mm; zwischen 299 mm und 329 mm, jedoch bevorzugt 314 mm; zwischen 329 mm und 359 mm, jedoch bevorzugt 344 mm. Es versteht sich, dass die Länge des Versteifungskörpers kürzer ist als die Länge des Stützkörpers.

Der Stützkörper oder/und der Versteifungskörper kann/können dazu eingerichtet sein, zumindest abschnittsweise einen Krümmungsradius zwischen 700 mm und 900 mm, jedoch bevorzugt von etwa 800 mm, aufzuweisen.

Figur 3a zeigt eine Draufsicht auf den Stützkörper 16, der eine Mehrzahl von Öffnungen 48 aufweist, die jeweils entlang des Stützkörpers 16 voneinander beabstandet im Wesentlichen mittig angeordnet sind. Figur 3b zeigt den Stützkörper 16 mit zwei daran angebrachten Versteifungskörpern 18, die mittels eines Sicherungselements 20 in Form einer Sicherungsklemme 50 an dem Stützkörper 16 angebracht sind. Die Klemme umgreift seitliche Abschnitte des Stützkörpers 16 und des Versteifungskörpers 18, sodass diese gegeneinander gedrückt und der Sicherungsklemme fixiert sind. Wie in Figur 3b ersichtlich, können mehrere Versteifungskörper 18 an einem Stützkörper 16 angebracht sein.

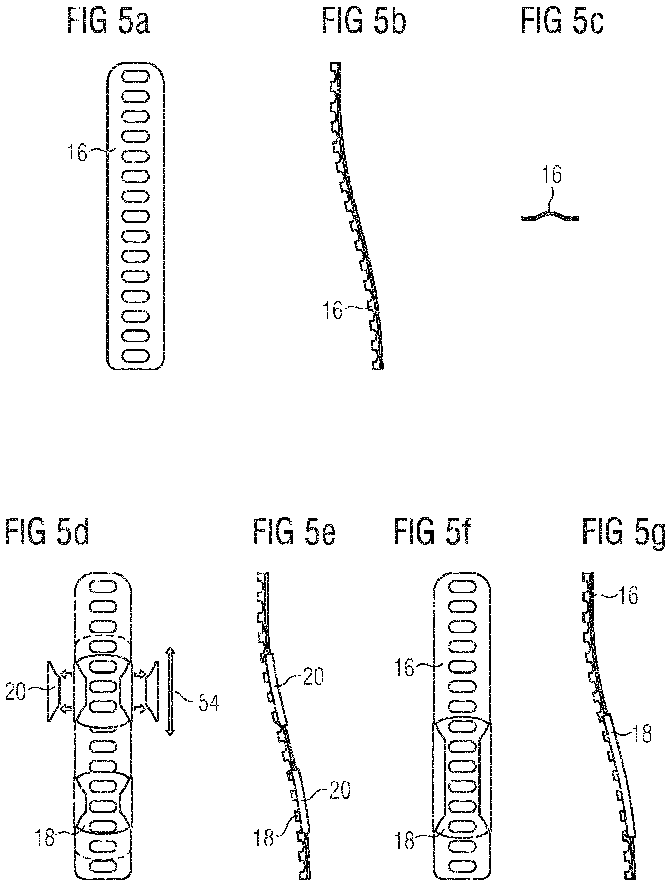

Die in Figuren 3a-b dargestellten Öffnungen 48 weisen eine längliche Form auf mit einer Längserstreckung zwischen 32 mm und 42 mm, jedoch bevorzugt von 37 mm, und einer Breitenerstreckung zwischen 11 mm und 21 mm, jedoch bevorzugt von 16 mm. Ferner ist in Figuren 3b und 5c ersichtlich, dass die Öffnungen 48 in einem Bereich des Stützkörpers oder/und des Versteifungskörpers angebracht sind, in dem dieser/diese eine Wölbung aufweisen. Mit anderen Worten, ein mittlerer Bereich entlang der der Längserstreckung des Stützkörpers oder/und des Versteifungskörpers ist konvex gewölbt, wobei die Öffnungen 48 im Bereich der Wölbung angeordnet sind.

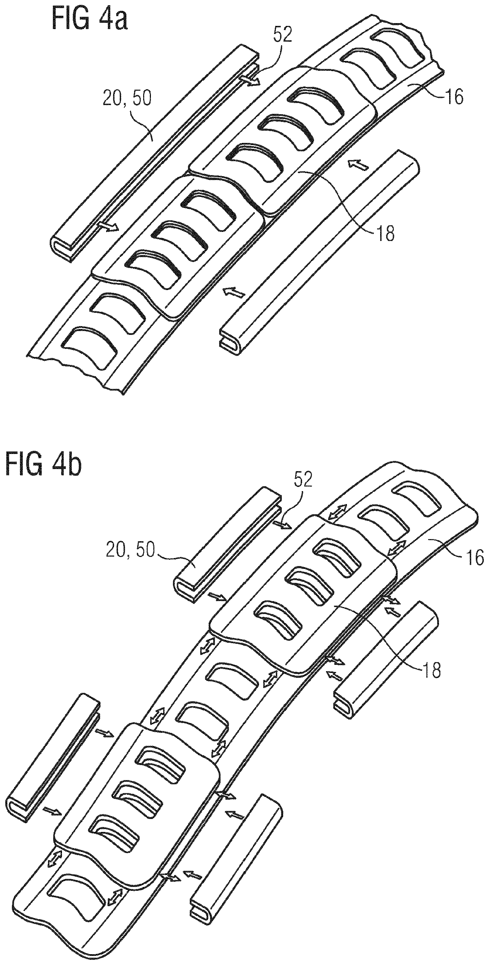

Figuren 4a-b zeigen in einer Explosionsansicht den Stützkörper 16, zwei Versteifungskörper 18 und entsprechende Sicherungsklemmen 50. Die Pfeile 52 in Figur 4a verdeutlichen, dass die Sicherungsklemme 50 seitlich auf die aneinander anliegenden Körper 16, 18 zur Fixierung dieser aneinander aufgeschoben werden.

Der Ablauf zur Sicherung des wenigstens einen Versteifungskörpers 18 an dem Stützkörper 16 ist in Figuren 5a-g dargestellt, wobei Figuren 5a, d und feine Draufsicht auf den Stützkörper 16, Figuren 5b, e und g eine Seitenansicht des Stützkörpers 16 und Figur 5c eine Frontansicht des Stützkörpers 16 zeigen. Ausgehend von dem anatomisch gebogenen Stützkörper 16 (Figur 5a-b) werden die Versteifungskörper 18 an gewünschten Positionen an dem Stützkörper 16 positioniert, wobei bei der Positionierung die Sicherungselemente 20 gelöst sind. Die Versteifungskörper 18 werden so lang in der durch den Pfeil 54 verdeutlichten Längsrichtung verschoben, bis sie an der gewünschten Position sind. An dieser werden die Sicherungselemente 20 befestigt (Figur 5d-e). Die Verbindung aus Stützkörper 16, Versteifungskörper 18 und Sicherungselement 20 ist in Figuren 5f-g dargestellt, wobei in diesen Darstellungen der Versteifungskörper 18 formschlüssig an dem Stützkörper 16 anliegt, beide Körper 16, 18 also im Verbindungsbereich eine identische Biegung aufweisen.

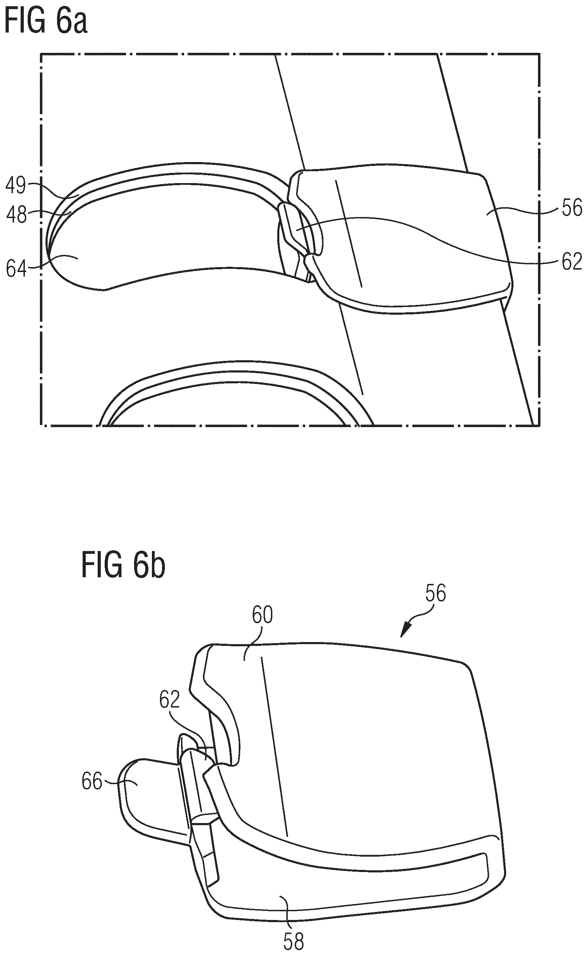

Das in Bezug auf Figuren 3a-b, 4a-b und 5a-g beschriebene Sicherungselement 20 ist in Form der Sicherungsklammer 50 ausgebildet. Alternativ kann das Sicherungselement ein anderes Verbindungselement sein, wie beispielsweise der in Figuren 6ab gezeigte Sicherungsclip 56. Der Sicherungsclip 56 umgreift, wie in Figur 6a dargestellt, den Stützkörper 16 und den Versteifungskörper 18 zumindest teilweise, sodass diese in einem Randbereich aneinandergedrückt werden. Dazu weist der Sicherungsclip 56 zwei elastisch oder/und schwenkbar miteinander verbundene Verbindungsabschnitte 58, 60 auf. Für eine bessere Sicherung des Stützkörpers 16 mit dem Versteifungskörper 18 weist der Sicherungsclip 56 einen Verrastabschnitt 62 auf, mittels welchem die Verbindungsabschnitte 58, 60 verbindbar sind. Wie in Figur 6a ersichtlich, umgreift der Sicherungsclip 56 mit dem Verrastabschnitt 62 und den zwei Verbindungsabschnitten 58, 60 einen jeweiligen Randbereich des Stützkörpers 16 und des Versteifungskörper 18 vollständig. Dabei ist eine Öffnung 48 des Stützkörpers 16 mit einer Öffnung 49 des Versteifungskörpers 18 derart ausgerichtet, dass sie gemeinsam eine Durchgangsöffnung 64 bilden, die der Sicherungsclip 56 durchtritt. Auf diese Weise verhindert der Sicherungsclip 56 zuverlässig eine axiale Verschiebung des Versteifungskörpers 18 gegenüber dem Stützkörper 16.

Vorzugsweise werden mindestens zwei, besser vier, Sicherungselemente 20 zur Sicherung eines Versteifungskörpers 18 an dem Stützkörper 16 verwendet. Mit der Verwendung von vier Sicherungsclips 56 kann zuverlässig ein Verschieben des Versteifungskörpers 18 gegenüber dem Stützkörper 16 verhindert werden. Ferner können dadurch ein seitliches Verdrehen sowie ein hierzu orthogonales Verkippen verhindert werden. Eine solche Verbindung ist beispielsweise in Figur 1a ersichtlich.

Die Sicherungselemente 20 sind so ausgebildet, dass diese wiederverwendbar sind, also mehrmals zur Sicherung verwendet werden können. Beispielsweise umfasst der Sicherungsclip 56 eine Öffnungshilfe 66 zum einfachen Öffnen und Schließen des Sicherungsclips 56. Die Sicherungselemente 20 weisen eine möglichst dünne Wandstärke auf, um zu verhindern, dass diese im Tragezustand den Benutzer stören, beispielsweise Druckstellen verursachen. Daher weisen die Sicherungselemente 20 eine geringere oder identische Wandstärke wie der Stützkörper 16 oder/und der Versteifungskörper 18 auf.

Ist der Stützkörper 16 mit der gewünschten Anzahl an Versteifungskörpern 18 verbunden, werden diese gemeinsam in eine Aufnahme in dem flexiblen Rückenelement 12 eingebracht. Die Aufnahme erlaubt nahezu keine Bewegung des Stützkörpers 16 in einer Längs- oder Querrichtung. Im Tragezustand liegt dann das flexible Rückenelement 12 an dem Rücken des Benutzers an, ist also dorsal angeordnet. Der Versteifungskörper 18 liegt wiederum dorsal an dem Stützkörper 16 an.

Es können mehrere Versteifungskörper 18 in einem Kit zusammen mit der Rückenorthese 10 zusammengestellt sein. Dies ermöglicht es, die gewünschte patienten- und behandlungsgerechte Steifigkeit der Verbindung aus Stütz- und Versteifungskörper 16, 18 bereitzustellen.

## Patentansprüche

1. Rückenorthese (10) zum Stützen der Lenden- oder/und Brustwirbelsäule, umfassend:
- ein flexibles Rückenelement (12), das dazu ausgelegt ist, in einem Tragezustand an einem Rücken eines Benutzers anzuliegen,
- einen Stützkörper (16), der an die menschliche Anatomie der Wirbelsäule angepasste oder anpassbare geschwungene Bereiche aufweist und in dem Rückenelement (12) aufgenommen ist, und
- ein Befestigungssystem (14), das mit dem Rückenelement (12) verbunden ist und dazu ausgelegt ist, in dem Tragezustand das Rückenelement (12) anpassbar an dem Benutzer anzubringen,
**dadurch gekennzeichnet, dass** die Rückenorthese (10) ferner wenigstens einen Versteifungskörper (18) umfasst, der verstellbar an dem Stützkörper (16) angebracht oder anbringbar ist.

2. Rückenorthese (10) nach Anspruch 1, wobei der Stützkörper (16) oder/und der wenigstens eine Versteifungskörper (18) elastisch verformbar ist/sind, sodass dieser/diese an eine jeweilige individuelle Krümmung der Wirbelsäule des Benutzers anpassbar ist/sind.

3. Rückenorthese (10) nach Anspruch 1 oder 2, wobei der wenigstens eine Versteifungskörper (18) dazu ausgelegt ist, flächig bzw. formschlüssig an einem Abschnitt des Stützkörpers (16) anzuliegen.

4. Rückenorthese (10) nach einem der vorhergehenden Ansprüche, wobei der wenigstens eine Versteifungskörper (18) dazu ausgelegt ist, mittels wenigstens einem Sicherungselement (20, 50, 56) an dem Stützkörper (16) gesichert zu sein.

5. Rückenorthese (10) nach Anspruch 4, wobei die Rückenorthese (10) wenigstens zwei Versteifungskörper (18) umfasst, die unabhängig voneinander oder miteinander verbunden an dem Stützkörper (16) angebracht oder anbringbar sind.

6. Rückenorthese (10) nach einem der vorhergehenden Ansprüche, wobei der Stützkörper (16) und der wenigstens eine Versteifungskörper (18) eine Mehrzahl an Öffnungen (48, 49) aufweisen, die jeweils entlang des Stützkörpers (16) voneinander beabstandet im Wesentlichen mittig angeordnet sind.

7. Rückenorthese (10) nach Anspruch 6, wobei der Stützkörper (16) und der wenigstens eine Versteifungskörper (18) derart gegeneinander verschiebbar sind, dass wenigstens eine Öffnung (48) des Stützkörpers (16) mit einer Öffnung (49) des wenigstens einen Versteifungskörpers (18) eine gemeinsame Durchgangsöffnung (64) bilden.

8. Rückenorthese (10) nach einem der Ansprüche 4 bis 6, wobei das wenigstens eine Sicherungselement (20, 50, 56) ein Verbindungselement (50, 56) oder eine Schraubvorrichtung umfassen.

9. Rückenorthese (10) nach Anspruch 8, wobei das Verbindungselement (50, 56) derart ausgebildet ist, dass es den Stützkörper (16) und den Versteifungskörper (18) seitlich umgreift oder/und die gemeinsame Durchgangsöffnung durchtritt, sodass der Stützkörper (16) und der wenigstens eine Versteifungskörper (18) aneinander gesichert sind.

10. Rückenorthese (10) nach einem der vorhergehenden Ansprüche, wobei das Befestigungssystem (14) zum Befestigen des Rückenelements (12) ein Gurtsystem aufweist, das einen ersten (24, 36, 40) und einen zweiten (26, 38, 42) Gurtstrang umfasst, wobei jeder der zwei Gurtstränge an zwei Befestigungsstellen und einer Umlenkstelle an dem Rückenelement (12) befestigt ist.

11. Rückenorthese (10) nach Anspruch 10, wobei
- eine erste der zwei Befestigungsstellen des ersten Gurtstrangs (24, 36, 40) an einem linken ersten Ende des Rückenelements (12) angeordnet ist, das im Tragezustand sinister ist, und
- eine zweite der zwei Befestigungsstellen des ersten Gurtstrangs (24, 36, 40) an einem linken zweiten Ende des Rückenelements (12) angeordnet ist, das im Tragezustand sinister ist.

12. Rückenorthese (10) nach Anspruch 10 oder 11, wobei jeder der beiden Gurtstränge (24, 26, 36, 38, 40, 42) wenigstens einen elastischen Abschnitt (28, 30, 43, 44) aufweist, wobei vorzugsweise ein jeweiliger mittlerer elastischer Abschnitt (43, 44) des ersten (24, 36, 40) bzw. zweiten (26, 38, 42) Gurtstranges in dem mittleren Bereich, wo der jeweilige Gurtstrang umgelenkt wird, angeordnet ist.

13. Rückenorthese (10) nach Anspruch 12, wobei ein jeweiliger elastischer Schulterabschnitt (28, 30) des ersten (24, 36, 40) bzw. zweiten (26, 38, 42) Gurtstranges in dem Tragezustand benachbart der Schulter eines Benutzers der Rückenorthese (10) angeordnet ist.

14. Rückenorthese (10) nach Anspruch 13, wobei jeder der elastischen Schulterabschnitte von einem jeweiligen Begrenzungszügel (32, 34) überspannt ist, der nicht elastisch sowie abnehmbar ist, sodass der jeweilige Gurtstrang im Bereich des linken bzw. rechten Schulterabschnitts nur dann flexibel ist, wenn der jeweilige Begrenzungszügel (32, 34) abgenommen ist.

15. Kit für eine Rückenorthese (10) nach einem der vorhergehenden Ansprüche, umfassend eine Mehrzahl an Versteifungskörpern (18) mit unterschiedlicher Steifigkeit oder/und unterschiedlicher Länge.
